# EUROPÄISCHE PATENTSCHRIFT

(11) **EP 1 502 626 B1**
(45) Veröffentlichungstag und Bekanntmachung des Hinweises auf die Patenterteilung: **07.08.2013**
(21) Anmeldenummer: 04026391.5
(22) Anmeldetag: 03.06.1998
(51) Int. Cl.: A61N 7/00, A61H 23/00

(54) **Medizinisches Instrument zur Behandlung von biologischem Gewebe**
Medical device for treating biological tissues
Appareil médical pour le traitement des tissues biologiques

(30) Priorität: 17.06.1997 DE 19725477
(43) Veröffentlichungstag der Anmeldung: 02.02.2005
(62) Teilanmeldung aus: 98934909.7
(73) Patentinhaber: Ferton Holding SA, 2800 Delemont (CH)
(72) Erfinder: Haupt, Gerald, 44577 Castrop (DE); Menne, Andreas, 1274 Signy (CH); Schulz, Manfred, 88662 Überlingen (DE)
(74) Vertreter: von Kreisler Selting Werner

(56) Entgegenhaltungen:
- EP-A- 0 317 507
- WO-A-92/09238
- WO-A-93/14720
- WO-A-93/16652
- FR-A- 1 062 415
- GB-A- 2 277 450
- US-A- 4 530 360
- US-A- 4 549 535
- US-A- 4 716 890

## Beschreibung

Die Erfindung betrifft ein medizinisches Instrument zur Behandlung von biologischem Gewebe.

Derartige Instrumente dienen dazu, mittels Druck- oder Stoßwellen den Heilungsprozeß bei Knochenbrüchen, Enthesiopathien, Tendopathien aber auch bei Parodontose zu beschleunigen. Ein weiteres Einsatzgebiet ist die Schmerztherapie im knochennahen Weichteilbereich des Haltungs- und Bewegungsapparates.

Bei bislang bekannten extrakorporalen Druckwellengeneratoren wird im Brennpunkt eines akustischen Reflektors, z.B. mittels einer Funkenentladung eine Druckoder Stoßwelle erzeugt, die dann durch den Reflektor auf das zu beschallende Objekt fokussiert wird. Es wird vermutet, daß mit Hilfe der Druckwellen Mikroschädigungen im biologischen Gewebe erzeugt werden, die den Körper zu Regenerationsmaßnahmen veranlassen.

Bekannte Druckimpulsquellen verwenden fokussierte Stoßwellen und können nur im eng begrenzten Fokusbereich eine Wirkung erzielen. Für ein befriedigendes Behandlungsergebnis muß allerdings der gesamte Knochenbruchbereich gleichmäßig beschallt werden. Dies verlangt einen aufwendigen Bewegungsmechanismus für die Druckimpulsquelle und ist zudem durch das wiederholte Aufsuchen der Behandlungspositionen sehr zeitintensiv.

In der Schmerztherapie tritt noch ein weiteres Problem bei der Verwendung bekannter Druckimpulsquellen auf. Die während der Behandlung eingesetzten Ortungssysteme zur Lokalisierung des Behandlungsortes (Ultraschall und Röntgen) können die Schmerzquelle nicht konkret anzeigen und der behandelnde Arzt beschallt mit einer großen Anzahl von Einzeilmpulsen den vermuteten Schmerzherd.

Aus der US 4,549,535 ist ein batteriebetriebenes Massagegerät bekannt, das eine klopfende Vibration ausführen soll. Ein solches Gerät hat die Aufgabe, auf der Hautoberfläche eine intensive Massagewirkung auszuüben. Zur Übertragung der mechanischen Impulse arbeitet das Gerät mit einem großen Hub der Sondenspitze. Des weiteren besteht die Sonde aus einer weichen Spitze aus Gummimaterial.

Die US 4,716,890 betrifft ein chiropraktisches Stoßgerät mit dem zur chiropraktischen Behandlung Stöße auf den Körper übertragen werden sollen. Auch bei diesem Gerät ist eine weiche, vorzugsweise aus Gummi, bestehende Spitze vorgesehen.

Die Geräte nach der US 4,549,535 und der US 4,716,890 sind daher lediglich geeignet, Stöße mit einem größeren Hub der Sonde auf den Körper zu übertragen und zwar zu Massagezwecken oder zu chiropraktischen Zwecken.

Der Erfindung liegt die Aufgabe zugrunde, ein medizinisches Instrument mit einem Druckwellengenerator so auszubilden, dass es auf eine einfache und kostengünstige Weise eine gleichmäßige Energieverteilung der Druckwellen auf einen großflächigen Wirkungsbereich ermöglicht.

Zur Lösung dieser Aufgabe dienen die Merkmale des Anspruchs 1.

Die Erfindung sieht in vorteilhafter Weise vor, daß das Übertragungselement eine stumpfe Sondenspitze mit einer flachen oder gekrümmten Austrittsfläche aufweist, die eine unfokussierte, mechanisch erzeugte Druckwelle mit hohen Druckspitzenwerten in das biologische Gewebe einkoppelt. Die Druckwelle kann sich dort bis zu ihrem Applikationsort ausbreiten, ohne daß das Übertragungselement in direktem Kontakt mit dem Applikationsort steht. Die Erfindung zielt darauf ab, die Druckwellen nicht zu fokussieren und es dadurch zu ermöglichen, daß diese großflächig eingekoppelt werden können. Das medizinische Instrument eignet sich insbesondere für Behandlungen, bei denen die Sondenspitze auf der Körperoberfläche sehr nah an dem Applikationsort angeordnet werden kann, wie dies beispielsweise bei einem Tennisellenbogen, einem Fersensporn oder auch bei Parodontose der Fall ist. Die Sondenspitze kann aufgrund der Kraftstöße auf die Eintrittsgrenzfläche des Übertragungselementes einen Hub von weniger als 0,5 mm ausführen.

Insbesondere bei orthopädischen Anwendungen ist es vorteilhaft, eine Vielzahl von einzelnen Druckwellen in das biologische Gewebe einzukoppeln, um eine optimale Wirkung zu erzielen. Das Antriebsmittel ist daher vorzugsweise so ausgestaltet, daß eine periodische Hin- und Herbewegung des Schlagteils möglich ist. Die Schlagfrequenz beträgt ca. 1 bis 30 Hz, vorzugsweise ca. 6 bis 20 Hz.

Das Übertragungselement ist axial und linear in dem Gehäuse geführt, wobei ein in Axialrichtung wirkendes Feder-Dämpfungselement zwischen dem Übertragungselement und dem Gehäuse angeordnet ist. Auf diese Weise wird eine Entkopplung des Übertragungselementes von dem Gehäuse in Axialrichtung realisiert. Außerdem bringt dieses Feder-Dämpfungselement das Übertragungselement wieder in seine Ausgangslage nach jeder Druckwelle zurück und begrenzt dessen Auslenkung. Für die Einkopplung der Druckwelle in das biologische Gewebe ist eine große Auslenkung der Ausgangsgrenzfläche des Übertragungselementes nicht notwendig. Die Einkopplung der Druckwelle soll sogar nach Möglichkeit nur aufgrund der Längenänderung des Übertragungselementes und nicht durch dessen Verlagerung erfolgen.

Bei einem weiteren Ausführungsbeispiel des Übertragungselementes kann vorgesehen sein, daß die Austrittsgrenzfläche einen erheblich größeren Durchmesser aufweist als die Eintrittsgrenzfläche. Eine derartige Austrittsgrenzfläche gewährleistet eine große Übertragungsfläche für die Einkopplung der Druckwelle, so daß die eingekoppelte spezifische Druckwellenenergie zur Schonung der Hautoberfläche reduziert ist.

Wesentliche Vorteile der Erfindung bestehen darin, daß das medizinische Instrument einen einfachen, kostengünstigen Aufbau aufweist, dessen Kosten lediglich einen Bruchteil bisher bekannter Druckwellengeneratoren beträgt.

Das medizinische Instrument kann als kleines transportables Gerät ausgebildet sein, das leichter applizierbar ist und ohne Behinderungen auf die zu behandelnde Körperstelle aufgesetzt werden kann. Das Gerät benötigt keine Verbrauchsmaterialien und insbesondere keine Ortungsvorrichtungen, da der Behandlungsbereich in der Nähe der Sondenspitze liegt.

Vorzugsweise ist vorgesehen, daß die Einrichtung zum Erzeugen der Druckwelle aus einem in einem Gehäuse geführten, mit Hilfe eines Antriebsmittels hin- und herbewegbaren Schlagteil besteht, das auf das Übertragungselement einen oder mehrere Kraftstöße ausübt, wobei das Schlagteil infolge des Kraftstoßes eine Druckwelle in das Übertragungselement induziert, die sich bis zu der Austrittsgrenzfläche der stumpfen Sondenspitze des Übertragungselementes fortpflanzt. Die Druckwelle wird demzufolge in einfacher Weise mechanisch erzeugt. Durch die schnelle Bewegung der Sondenspitze läßt sich eine Druckwelle mit hohen Druckspitzenwerten erzeugen. Die Druckwelle pflanzt sich in dem biologischen Gewebe fort und wird nicht fokussiert. Die mit einem solchen System erzeugten Druckwellen erreichen nicht die kurzen Anstiegszeiten der Druckwellengeneratoren mit fokussierter Druckwelle, sind aber in der maximalen Druckspitze nicht wesentlich schwächer. Die unfokussierte Druckwelle breitet sich bis zum Anwendungsort in dem biologischen Gewebe radial aus.

Das Schlagteil ist vorzugsweise koaxial zu dem Übertragungselement angeordnet. Auf diese Weise kommt es zu einem direkten Impulsaustausch zwischen dem Schlagteil und dem Übertragungselement beim Aufschlagen des Schlagteils auf die Eintrittsgrenzfläche des Übertragungselementes.

Zwischen dem Schlagteil und dem Übertragungselement kann ein Zwischenelement angeordnet sein, das den Kraftstoß von dem Schlagteil auf das Übertragungselement weiterleitet. Dieses Zwischenelement kann dazu dienen, eine bessere Abschirmung der Antriebsmittel gegenüber dem Applikationsbereich zu schaffen oder zum Umlenken der Richtung der Druckwelle oder auch zum Beeinflussen der Druckwellencharakteristik dienen.

An dem proximalen Ende der Führung des Schlagteils kann ein Magnethalter für das Schlagteil angeordnet sein, der das Schlagteil in der proximalen Endlage hält, bis es von den Antriebsmitteln erneut beschleunigt wird.

Die stumpfe Sondenspitze weist vorzugweise eine flache Austrittsgrenzfläche mit abgerundeten Kanten auf. Die Austrittsgrenzfläche des Übertragungselementes ist möglichst großflächig, um einen hohen Wirkungsgrad bei der Übertragung der Druckwelle zu erzielen. Die gerundeten Kanten vermeiden Verletzungen der Hautoberfläche.

Die Sondenspitze kann auch eine Austrittsgrenzfläche aufweisen, die konvex gekrümmt ist und ebenfalls mit gerundeten Kanten versehen ist.

Zwischen der Sondenspitze und der Einkoppelstelle auf dem biologischen Gewebe kann ein Impedanzanpassungsmedium angeordnet sein, das die Einkopplung der Druckwelle in das biologische Gewebe verbessert. Ein geeignetes pastenförmiges Impedanzanpassungsmedium ist beispielsweise ein Ultraschallgel oder eine andere pastöse Masse, z.B. Vaseline.

Das Übertragungselement kann zur Impedanzanpassung aus unterschiedlichen Materialien aufgebaut sein, die das Übertragungsverhalten verbessern. Durch eine geeignete Materialauswahl kann das Übertragungsverhalten der Druckwelle bzw. des Übertragungselementes und damit die Einkopplung in das biologische Gewebe beeinflußt werden. Das einstückige Übertragungselement aus unterschiedlichen Materialien aufzubauen, hat das Ziel, die Druckwelle möglichst verlustarm in den Körper einzukoppeln, und dadurch eine Impedanzanpassung zu ermöglichen.

Die Länge des Übertragungselementes kann im Bereich zwischen ca. 20 und 100 mm liegen. Über unterschiedliche und auswechselbare Übertragungselemente kann eine Anpassung an die gewünschte Behandlung erfolgen.

Im folgenden werden unter Bezugnahme auf die Zeichnungen Ausführungsbeispiele der Erfindung näher erläutert.

Es zeigen:
- Fig. 1: eine Darstellung des medizinischen Instrumentes im Querschnitt, und
- Fign.2 und 3: alternative Ausführungsformen des Übertragungselementes

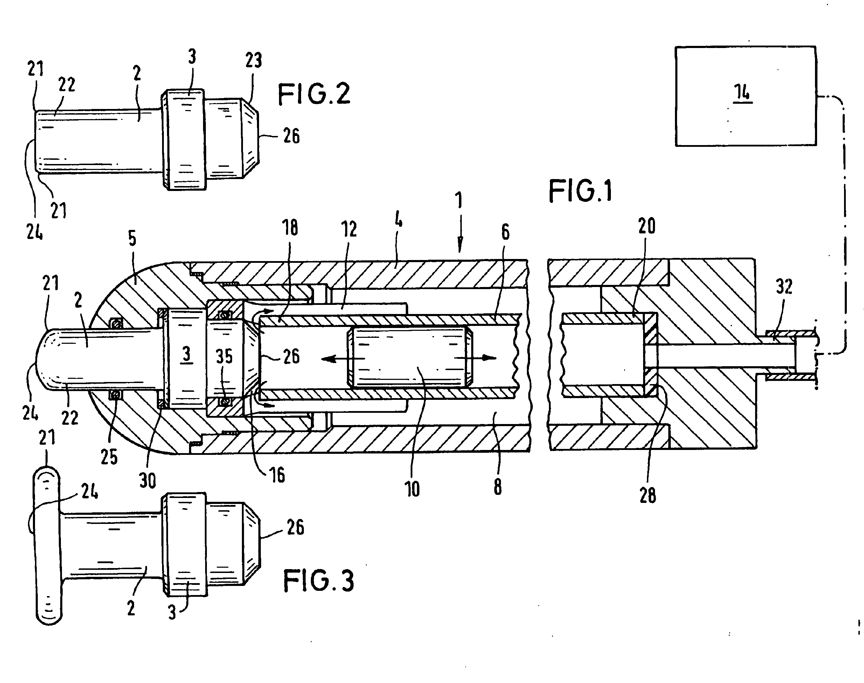

Das in Fig. 1 gezeigte Handstück 1 besteht aus einem Gehäuse 4, das einen pneumatischen Zylinder 6 aufnimmt, in dem ein Schlagteil 10 mit Hilfe pneumatischer Antriebsmittel 14 in Verbindung mit einer Staudruckkammer 8, die den Zylinder 6 koaxial ringförmig umgibt, zwischen zwei Endpositionen hin- und herbewegt wird. Der Beschleunigungsweg weist bei einem pneumatischen Antrieb ca. 100 bis 200 mm Länge auf.

In der proximalen Endposition des Schlagteils 10 ist am proximalen Ende 20 des Zylinders 6 ein Magnethalter 28 angeordnet, der das metallische Schlagteil 10 in seiner proximalen Endposition festhalten kann bis erneut ein über den Anschluß 32 aufgebrachter pneumatischer Druck das Schlagteil 10 in Richtung auf das distale Ende 18 des Zylinders 6 beschleunigt. Die sich in Bewegungsrichtung des Schlagteils 10 vor dem Schlagteil befindliche Luft wird über einen an dem distalen Ende 18 des Zylinders 6 befindlichen Ringschlitz 16 in die Staudruckkammer 8 geleitet. Durch die Beschleunigung des Schlagteils 10 trifft dieses mit einer hohen Endgeschwindigkeit von beispielsweise 5 bis 20 m/s auf eine distal von dem Zylinder 6 angeordnete Eintrittsgrenzfläche 26 eines Übertragungselementes 2. Das Übertragungselement 2 besteht aus einer metallischen Sonde, mit einer stumpfen Sondenspitze 22 und einer planen oder konvex geformten Austrittsgrenzfläche 24. Das Schlagteil 10 übt einen oder mehrere Kraftstöße auf das Übertragungselement 2 aus, das die von dem Schlagteil 10 induzierte Druckwelle an die Austrittsgrenzfläche 24 weiterleitet und dort in ein biologisches Gewebe einkoppelt.

Das Übertragungselement 2 ist in dem Gehäuse 4 linear und vorzugsweise koaxial zu dem Schlagteil 10 geführt. Das Gehäuse 4 weist ein Kopfteil 5 auf, das für ein Auswechseln des Übertragungselementes 2 abschraubbar ist. Das Übertragungselement 2 ist in einer Bohrung des Kopfteils 5 gelagert und mit Hilfe eines O-Rings 25 im vorderen Abschnitt des Kopfteils 5 abgedichtet. Ein Ringbund 3 des Übertragungselementes 2 dient als Anschlagelement wobei zwischen dem Ringbund 3 des Übertragungselementes 2 und dem Kopfteil 5 des Gehäuses 4 ein Feder/Dämpfungselement 30 angeordnet ist, das das Übertragungselement 2 von dem Gehäuse 4 in Axialrichtung entkoppelt. Das Feder/Dämpfungselement 30 hat aber auch eine Rückstellfunktion für das Übertragungselement 2 und drückt dieses nach jeder Druckwelle in seine proximale Ausgangslage zurück. Zugleich begrenzt es dessen Verlagerung während einer Druckwelle. Für die Einkopplung der Druckwelle in das biologische Gewebe ist eine Verlagerung des Übertragungselementes nicht notwendig und sogar unerwünscht, um Verletzungen zu vermeiden. Die Druckwelle soll allein durch die Längenänderung des Übertragungselementes 2 infolge der Druckwelle in das biologische Gewebe eingekoppelt werden.

Der sich in der Staudruckkammer 80 aufbauende Staudruck genügt bei Wegfall des an dem pneumatischen Anschluß 32 anliegenden Druckes, um das Schlagteil 10 von der distalen Endlage an dem Übertragungselement 2 in die proximale Endlage zu dem Magnethalter 28 zurückzubewegen. Der pneumatische Druck an dem Anschluß 32 kann bis zu 5 bar betragen. Das Schlagteil 10 kann zwecks Anpassung an bestimmte Längen des Übertragungselementes 2 oder zum Erzeugen einer bestimmten Charakteristik der Druckwelle hinsichtlich Länge, Masse und maximale Aufschlaggeschwindigkeit unterschiedlich gewählt werden.

Die proximale Eintrittsgrenzfläche 26 des Übertragungselementes 2 weist in etwa den gleichen Druchmesser wie das Schlagteil 10 auf. Dagegen kann die Austrittsgrenzfläche 24 einen beispielsweise um den Faktor 2 größeren Durchmesser aufweisen als die Eintrittsgrenzfläche 26. Die Länge des Schlagteils 10 ist vorzugsweise größer als sein Durchmesser. Damit werden bessere Führungseigenschaften in dem Zylinder 6 erreicht. Außerdem kann mit Hilfe einer unterschiedlichen Länge Durchmesser des Zylinders 6 und der Eintrittsgrenzfläche 26 des Übertragungselementes 2 verändert werden müssen.

Das proximale Ende des Übertragungselementes 2 ist in einer geschlitzten Hülse 12 geführt und in dieser mit Hilfe eines O-Rings 35 radial abgedichtet. Die Hülse 12 bildet mit dem proximalen konischen Ende des Übertragungselementes 2 eine Verbindung zwischen der Staudruckkammer 8 und dem distal von dem Schlagteil 10 liegenden Hohlraum des Zylinders 6.

Die Druckwellen werden mit einer Schlagfrequenz von ca. 1 bis 30 Hz, vorzugsweise 6 bis 20 Hz, erzeugt. Die Sondenspitze 22 führt einen Bewegungshub aus, der weniger als 0,5 mm beträgt.

Fig. 2 zeigt ein Übertragungselement 2 mit einer flachen, stumpfen Sondenspitze 22 mit abgerundeten Kanten.

Bei dem Ausführungsbeispiel des Übertragungselementes 2 gemäß Fig. 3 ist die Austrittsgrenzfläche 24 im Vergleich zur Eintrittsgrenzfläche 26 erheblich vergrößert. Das Durchmesserverhältnis der Austrittsgrenzfläche 24 zu der Eintrittsgrenzfläche 26 beträgt dabei ca. 2 bis 3.

Das medizinische Instrument ermöglicht es, ein therapeutisches Verfahren zum Behandeln von biologischem Hart- und Weichgewebe, insbesondere zur Heilung von Knochenleiden, wie Knochenbrüchen, Enthesiopathien, Tendopathien und Parodontose, sowie eine Schmerztherapie im knochennahen Weichteilbereich des Haltungs- und Bewegungsapparates, durchzuführen, indem mechanisch erzeugte Druckwellen unfokussiert über eine stumpfe Sonde mit flacher Austrittsgrenzfläche in das biologische Gewebe eingekoppelt werden.

Das Übertragungselement 2 kann aus unterschiedlichen Materialien bestehen, um die Druckwelle möglichst verlustarm in das biologische Gewebe einzukoppeln und dadurch eine Impedanzanpassung zu erzielen. Dabei kann vorgesehen sein, daß die unterschiedlichen Materialien in Axialrichtung hintereinander angeordnet sind.

## Patentansprüche

1. Medizinisches Instrument zur Behandlung von biologischem Gewebe eines Körpers von Lebewesen,
mit einem Gehäuse (4),
mit einem Antriebsmittel (14) zum Beschleunigen eines Schlagteils (10) zum Erzeugen von Druckwellen auf ein Übertragungselement (2), wobei
das Schlagteil (10) für ein wiederholtes Anschlagen gegen das Übertragungselement (2) in dem Gehäuse (4) periodisch hin- und herbewegbar ist und selbsttätig rückstellbar ist,
das Antriebsmittel (14) das Schlagteil (10) pneumatisch mit einem Druck von bis zu 5 bar beschleunigt,
das Schlagteil (10) über das Übertragungselement (2) unfokussierte, mechanisch erzeugte Druckwellen in das biologische Gewebe einkoppelt,
das Übertragungselement (2) aus einer Sonde besteht, deren stumpfe Sondenspitze eine flache oder gekrümmte Austrittsgrenzfläche (24) aufweist,
die Sondenspitze (22) zur Einkopplung der Druckwellen auf der Körperoberfläche geeignet ist,
zwischen Übertragungselement (2) und dem Gehäuse (4) ein in Axialrichtung wirkendes federndes Dämpfungselement (30) angeordnet ist, gegen das ein Ringbund (3) des Übertragungselementes (2) als Anschlagelement anliegt,
das Dämpfungselement (30) das Übertragungselement (2) in Axialrichtung von dem Gehäuse (4) entkoppelt,
das Dämpfungselement (30) die Verlagerung des Übertragungselementes (2) begrenzt, so dass die Sondenspitze (22) einen Hub von weniger als 0,5 mm ausführt,
und wobei
die Schlagfrequenz des Schlagteils (10) 1 bis 30 Hz, vorzugsweise 6 bis 20 Hz beträgt.

2. Medizinisches Instrument nach Anspruch 1, **dadurch gekennzeichnet, dass** das Schlagteil (10) mit Hilfe des Antriebsmittels (14) in Verbindung mit einer Staudruckkammer (8) zwischen zwei Endpositionen hin- und herbewegbar ist.

3. Medizinisches Instrument nach einem der Ansprüche 1 bis 2, **dadurch gekennzeichnet, dass** zwischen Schlagteil (10) und dem Übertragungselement (2) ein Zwischenelement angeordnet ist, das den Kraftstoß von dem Schlagteil (10) auf das Übertragungselement (2) weiterleitet.

4. Medizinisches Instrument nach einem der Ansprüche 1 bis 3, **dadurch gekennzeichnet, dass** an dem proximalen Ende (20) der Führung (6) des Schlagteils (10) ein Magnethalter (28) für das Schlagteil (10) angeordnet ist.

## Claims

1. Medical instrument for treating biological tissue of a body of a living organism, comprising
a housing (4),
an actuating means (14) for accelerating a beater portion (10) to generate pressure waves acting on a transmission element (2), wherein
the beater portion (10) is capable of being periodically reciprocated in the housing (4) for the purpose of repeatedly striking onto the transmission element (2) and is capable of being reset automatically,
the actuating means (14) accelerates the beater portion (10) pneumatically with a pressure up to 5 bar,
the beater portion (10) couples non-focused mechanically generated pressure waves into the biological tissue via the transmission element (2),
the transmission element (2) is formed by a probe which has a blunt probe tip with a flat or curved exit interface (24),
the probe tip (22) is suitable for coupling in the pressure waves on the body surface,
between the transmission element (2) and the housing (4), a resilient damping element (30) acting in the axial direction is arranged, against which abuts an annular collar (3) of the transmission element (2) as an abutment element,
the damping element (30) decouples the transmission element (2) from the housing (4) in the axial direction,
the damping element (30) restricts the displacement of the transmission element (2), so that the probe tip (22) performs a stroke of less than 0.5 mm,
and wherein
the beating frequency of the beater portion (10) is 1 to 30 Hz, preferably 6 to 20 Hz.

2. The medical instrument of claim 1, **characterized in that** the beater portion (10) is capable of being reciprocated between two end positions with the aid of the drive means (14) in combination with a dynamic pressure chamber (8).

3. The medical instrument of one of claims 1 to 2, **characterized in that** an intermediate element is arranged between the beater portion (10) and the transmission element (2), which intermediate element transmits the impulse from the beater portion (10) to the transmission element (2).

4. The medical instrument of one of claims 1 to 3, **characterized in that** a magnetic holder (28) for the beater portion (10) is arranged at the proximal end (20) of the guide (6) of the beater portion (10).

## Revendications

1. Instrument médical pour le traitement du tissu biologique d'un corps d'être vivant,
avec un boîtier (4),
avec un moyen d'entraînement (14) pour l'accélération d'une partie de percussion (10) pour la production d'ondes de pression sur un élément de transmission (2),
la partie de percussion (10) pouvant, pour une percussion répétée contre l'élément de transmission (2), être déplacée dans le boîtier (4) périodiquement dans un sens et dans l'autre et pouvant être rappelée automatiquement,
le moyen d'entraînement (14) accélérant la partie de percussion (10) pneumatiquement avec une pression pouvant atteindre 5 bars,
la partie de percussion (10) injectant dans le tissu biologique, par le biais de l'élément de transmission (2), des ondes de pression non focalisées et produites mécaniquement,
l'élément de transmission (2) se composant d'une sonde dont la pointe de sonde non pointue présente une surface limite de sortie (24) plate ou courbe,
la pointe de sonde (22) étant appropriée pour l'injection des ondes de pression sur la surface du corps,
un élément amortisseur (30) élastique agissant dans la direction axiale étant disposé entre l'élément de transmission (2) et le boîtier (4), un collet rond (3) de l'élément de transmission (2) appuyant, en tant qu'élément de butée, contre l'élément amortisseur,
l'élément amortisseur (30) opérant dans la direction axiale un découplage entre l'élément de transmission (2) et le boîtier (4),
l'élément amortisseur (30) limitant le déplacement de l'élément de transmission (2) de telle sorte que la pointe de sonde (22) effectue une course de moins de 0,5 mm,
la fréquence de percussion de la partie de percussion (10) étant de 1 à 30 Hz, de préférence de 6 à 20 Hz.

2. Instrument médical selon la revendication 1, **caractérisé en ce que** la partie de percussion (10) peut être déplacée dans un sens et dans l'autre entre deux positions extrêmes à l'aide du moyen d'entraînement (14) en lien avec une chambre de pression dynamique (8).

3. Instrument médical selon l'une des revendications 1 à 2, **caractérisé en ce que**, entre la partie de percussion (10) et l'élément de transmission (2), il est disposé un élément intermédiaire qui transmet l'impulsion depuis la partie de percussion (10) vers l'élément de transmission (2).

4. Instrument médical selon l'une des revendications 1 à 3, **caractérisé en ce que**, à l'extrémité proximale (20) du guide (6) de la partie de percussion (10), il est disposé un support magnétique (28) pour la partie de percussion (10).
